(19) Europäisches Patentamt
European Patent Office
Office européen des brevets

(11) **EP 1 668 349 B1**

(12) **FASCICULE DE BREVET EUROPEEN**

(45) Date de publication et mention de la délivrance du brevet:
**15.08.2007 Bulletin 2007/33**

(21) Numéro de dépôt: **04787361.7**

(22) Date de dépôt: **13.09.2004**

(51) Int Cl.:
*G01N 27/403* (2006.01)   *G01N 33/487* (2006.01)

(86) Numéro de dépôt international:
**PCT/FR2004/002315**

(87) Numéro de publication internationale:
**WO 2005/026714 (24.03.2005 Gazette 2005/12)**

(54) **CELLULE DE MESURE D'ACTIVITES BIOLOGIQUES ET/OU DE GRANDEURS PHYSIOLOGIQUES DE MICRO-ORGANISMES**

ZELLE ZUR MESSUNG BIOLOGISCHER AKTIVITÄTEN UND/ODER PHYSIOLOGISCHER PARAMETER VON MIKROORGANISMEN

CELL FOR MEASURING BIOLOGICAL ACTIVITIES AND/OR PHYSIOLOGICAL PARAMETERS OF MICRO-ORGANISMS

(84) Etats contractants désignés:
**AT BE BG CH CY CZ DE DK EE ES FI FR GB GR HU IE IT LI LU MC NL PL PT RO SE SI SK TR**

(30) Priorité: **11.09.2003 FR 0310704**

(43) Date de publication de la demande:
**14.06.2006 Bulletin 2006/24**

(73) Titulaire: **Université de Bourgogne
21078 Dijon Cedex (FR)**

(72) Inventeurs:
• **CACHON, Rémy**
  **F-21000 Dijon (FR)**
• **WACHE, Yves**
  **F-21640 Vougeot (FR)**
• **ALWAZEER, Duried,**
  **Al-Boath University**
  **Homs (SY) (SY)**
• **RIONDET, Christophe**
  **F-21850 Saint-Appollinaire (FR)**
• **GUYONDET, Patrick**
  **F-21300 Chenove (FR)**

(74) Mandataire: **Honoré, Anne-Claire et al
Cabinet Claude Guiu
10, rue Paul Thénard
21000 Dijon (FR)**

(56) Documents cités:
EP-A- 0 514 575       FR-A- 1 356 645
US-A- 3 471 393       US-A- 5 254 461
US-A- 5 571 396       US-A- 6 096 275

• **DATABASE WPI 1991, Derwent Publications Ltd., London, GB; AN 1992-158551 XP002277572 KHOLMUKHAMEDOV: "liquid medium testing device" & SU 1 656 438 A (AS USSR BIOL PHYS) 15 juin 1991 (1991-06-15) cité dans la demande**

## Description

La présente invention concerne un dispositif pour la mesure en continu d'activités biologiques et/ou de grandeurs physiologiques telles que le pH intracellulaire, le gradient de pH ($\Delta$pH), le gradient électrique transmembranaire ($\Delta\Psi$), la force proton motrice, l'activité enzymatique ou analogues, de cellules telles que des bactéries ou des enzymes isolées par exemple et/ou pour la culture de petits volumes de micro-organismes.

Il est bien connu de mesurer le pH intracellulaire ($pH_{in}$) de micro-organismes tels que des bactéries compte tenue du rôle du pH intracellulaire dans les diverses fonctions physiologiques vitales des cellules de ces micro-organismes. Afin de mesurer le $pH_{in}$ de micro-organismes, on connaît bien des méthodes dites des indicateurs de pH tels que l'indicateur $^{31}$p de spectroscopie à résonance magnétique nucléaire (NMR) et les sondes fluorescentes pH. Le procédé de mesure à indicateur de pH tel que l'indicateur $^{31}$p (NMR) est peu utilisé par les chercheurs compte tenu de sa complexité et du coût excessif du matériel nécessaire pour la mise en oeuvre de ce procédé. Par ailleurs, la méthode des sondes fluorescentes ne permet pas de mesurer le $pH_{in}$ de certaines bactéries notamment. En effet, la valeur du $pH_{in}$ de différentes bactéries peut être comprise entre 5,6 et 9 de sorte que, aucune sonde n'étant susceptible de couvrir cette plage de valeur, il est nécessaire d'utiliser un grand nombre de sondes grevant ainsi considérablement le coût de cette méthode. De plus, les sondes fluorescentes présentent une faible rétention à l'intérieur de la cellule chez certaines bactéries, rendant ainsi la méthode totalement inefficace. On observera, par ailleurs, que ce type de procédé présente l'inconvénient de ne pas permettre un contrôle de l'environnement dans lequel sont positionnées les cellules analysées.

Afin de remédier à ces inconvénients, on a déjà imaginé une méthode dite de "distribution des ions" qui est basée sur l'hypothèse que la forme neutre lipophil de la sonde est seulement perméable à travers la membrane cytoplasmique des cellules des micro-organismes et que la forme ionisée hydrophil est imperméable. Ainsi, quand le milieu intérieur de la cellule est alcalin par rapport au milieu extérieur, la forme neutre de la sonde pénètre la membrane cellulaire des micro-organismes et se dissout dans le cytoplasme en fonction de son pKa et du pH cytoplasmique jusqu'à l'état d'équilibre. Le flux du milieu extérieur vers l'intérieur à travers la membrane de la cellule est détecté et mesuré par une électrode sensible à la forme neutre lipophil de la sonde qui mesure la concentration en mesurant une force électromotrice, de l'ordre du millivolt, entre une électrode obtenue dans du platine ou de l'or et une électrode de référence immergée dans le milieu à mesurer, ladite force électromotrice se manifestant par un potentiel électrique qui est générée par l'électrode et mesuré par des moyens de traitements des signaux électriques.

Ainsi, on a déjà imaginé des cellules de mesure telles que la cellule mise au point par l'Université néerlandaise de Groningen dans les laboratoires du professeur Konings comportant une sonde pour mesurer le potentiel de membrane des micro-organismes communément noté $\Delta\Psi$, une sonde de référence dite électrode "au calomel" et une sonde pour la mesure du gradient $\Delta$pH débouchant dans une chambre apte à recevoir les micro-organismes à analyser, lesdites électrodes étant connectées à des moyens de mesure et des moyens de traitement des signaux électriques émis par les électrodes.

Par ailleurs, on a déjà imaginé des dispositifs servant à la mesure d'activité biologique et/ou grandeurs physiologiques comprenant une cellule de mesure pourvue d'une chambre apte à recevoir des cellules à analyser du type enzyme ou micro-organismes, et d'une ou plusieurs sondes qui débouche dans la chambre où les sondes sont connectées à des moyens de mesure et de traitement, et où la cellule de mesure présente un puits vertical ouvert dans sa partie supérieure et fermée dans sa partie inférieure. La paroi latérale du puits est pourvue de trous afin que les sondes débouchent à l'intérieur du puits, lesdites sondes étant solidarisées au puits de manière étanche et s'étendant dans des moyens de support répartis autour du puits. C'est le cas par exemple du brevet américain n°6 096 275 ou du brevet provenant de l'Union Soviétique n° SU 16..56438.

En outre le brevet français FR 2 779 525 décrit un appareil comprenant essentiellement un support dans lequel est placée une carte de mesure, un dispositif de perfusion, un thermostat, un boîtier de mesure et un système de gestion des mesures. La carte de mesure comprend un réseau d'électrodes dont une extrémité débouche dans une chambre et dont l'autre extrémité est placée en dehors de cette chambre. Les micro-organismes sont placés dans la chambre qui est connectée au dispositif de perfusion et le support comporte un connecteur reliant les électrodes de la carte au système de gestion des mesures par l'intermédiaire des boîtiers de mesure permettant d'effectuer des mesures en continu, et accessoirement à distance au moyen d'un modem.

Néanmoins, tous ces dispositifs présentent l'inconvénient d'être difficilement nettoyables de sorte que des bactéries d'une précédente expérimentation sont susceptibles de fausser les mesures de l'expérimentation suivante.

Par ailleurs, les bioréacteurs présentent l'inconvénient de nécessiter une mise en culture des micro-organismes à étudier dans un grand volume souvent inutile pour des travaux de recherche. De plus, on observera que les bioréacteurs présentent un ratio surface/volume particulièrement faible de sorte que la température n'est pas homogène dans le bio réacteur en cas de choc thermique ce qui risque de fausser les résultats de l'expérience.

L'un des buts de l'invention est donc de remédier à tous ces inconvénients en proposant un dispositif pour la mesure d'activités biologiques et/ou de grandeurs physiologique de conception simple et peu onéreuse permettant, outre les mesures, de mettre en culture des micro-organismes dans un petit volume.

A cet effet et conformément à l'invention, il est proposé un dispositif pour la mesure de grandeurs physiologiques telles que le pH intracellulaire, le pH extracellulaire, la force proton motrice, l'activité enzymatique ou analogues, comprenant une cellule dite de mesure pourvue d'une chambre apte à recevoir les micro-organismes à analyser et une ou plusieurs sondes débouchant dans la chambre, lesdites sondes étant connectées à des moyens de mesure et des moyens de traitement des signaux électriques émis par les électrodes remarquable en ce que la cellule de mesure comporte un puits vertical de section transversale quelconque, ouvert à son extrémité supérieure et fermé à son extrémité inférieure obtenu dans du polychlorure de vinyle (PVC), muni sur sa paroi latérale de trous uniformément répartis autour du puits pour permettre aux sondes de déboucher à l'intérieur du puits, lesdites sondes étant solidarisées de manière étanche au puits et s'étendant dans des moyens de support uniformément répartis autour du puits et solidaires dudit puits, et apte à recevoir une cuvette de section transversale homothétique à la section du puits et munie sur sa paroi latérale de trous qui s'étendent au droit des trous du puits lorsque la cuvette est positionnée dans le puits pour permettre aux sondes de déboucher à l'intérieur de ladite cuvette dans laquelle sont placés les miro-organismes à analyser, ladite cuvette étant réalisée dans un matériau métallique amagnétique susceptible de subir une stérilisation sans détérioration.

D'autres avantages et caractéristiques ressortiront mieux de la description qui va suivre du dispositif pour la mesure d'activités biologiques et/ou de grandeurs physiologiques de micro-organismes conforme à l'invention en référence aux dessins annexés sur lesquels :

- la figure 1 est une vue en perspective schématique du dispositif pour la mesure d'activités biologiques et/ou de grandeurs physiologiques de micro-organismes conforme à l'invention,
- la figure 2 est une vue en coupe transversale suivant la ligne de coupe II-II' de la figure 1 du dispositif suivant l'invention,
- la figure 3 est une vue en coupe radiale suivant la ligne III-III' de la figure 2 du dispositif suivant l'invention,
  La figure 4 est une vue en coupe longitudinale d'une sonde du dispositif suivant l'invention,
- la figure 5 est une vue longitudinale du fourreau d'une sonde du dispositif suivant l'invention,
- la figure 6 est une vue en coupe longitudinale d'une seconde partie du fourreau de la sonde du dispositif suivant l'invention,
- la figure 7 est une vue en coupe longitudinale de la membrane positionnée à l'extrémité distale de la sonde du dispositif suivant l'invention,
- la figure 8 est une vue en coupe longitudinale d'une électrode constituant la sonde du dispositif conforme à l'invention.

On décrira dans cet exemple de réalisation non limitatif, un dispositif pour la mesure d'activités biologiques et/ou de grandeurs physiologiques telles que le pH intracellulaire, le pH extracellulaire, le gradient de pH ($\Delta$pH), la force proton motrice, l'activité enzymatique ou analogues de micro-organismes tels que des bactéries, par exemple ; néanmoins, le dispositif pourra être adapté à d'autres types de micro-organismes et à d'autres types de mesure, sans pour autant sortir du cadre de l'invention.

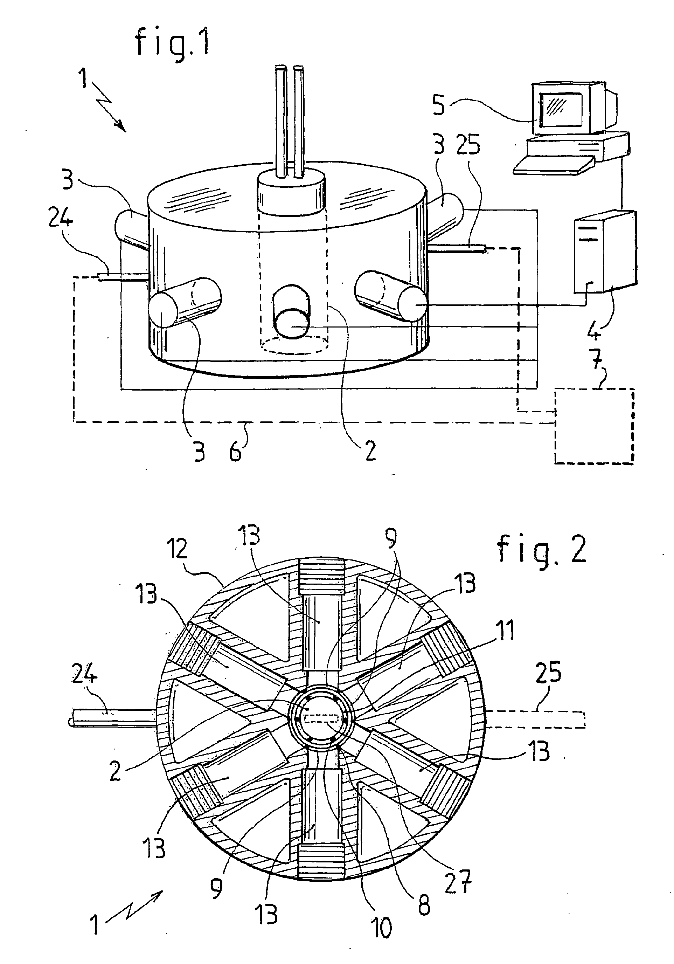

En référence à la figure 1, le dispositif suivant l'invention comprend une cellule dite de mesure 1 pourvue d'une chambre 2 apte à recevoir les micro-organismes à analyser et une ou plusieurs sondes 3 débouchant dans la chambre 2, lesdites sondes 3 étant connectées à des moyens de mesure 4 et des moyens de traitement 5 des signaux électriques émis par les sondes 3. Les moyens de mesure 4 et les moyens de traitement 5 peuvent consister, par exemple, en une interface électronique et respectivement un logiciel enregistré sur un ordinateur du type PC. De plus, le dispositif comporte avantageusement des moyens de chauffage des micro-organismes placés dans la chambre 2 de la cellule de mesure 1 consistant, par exemple, en un circuit 6 de fluide caloporteur à la périphérie de la chambre 2 de la cellule de mesure 1 alimenté par des moyens de régulation thermique 7, comme il sera détaillé plus loin.

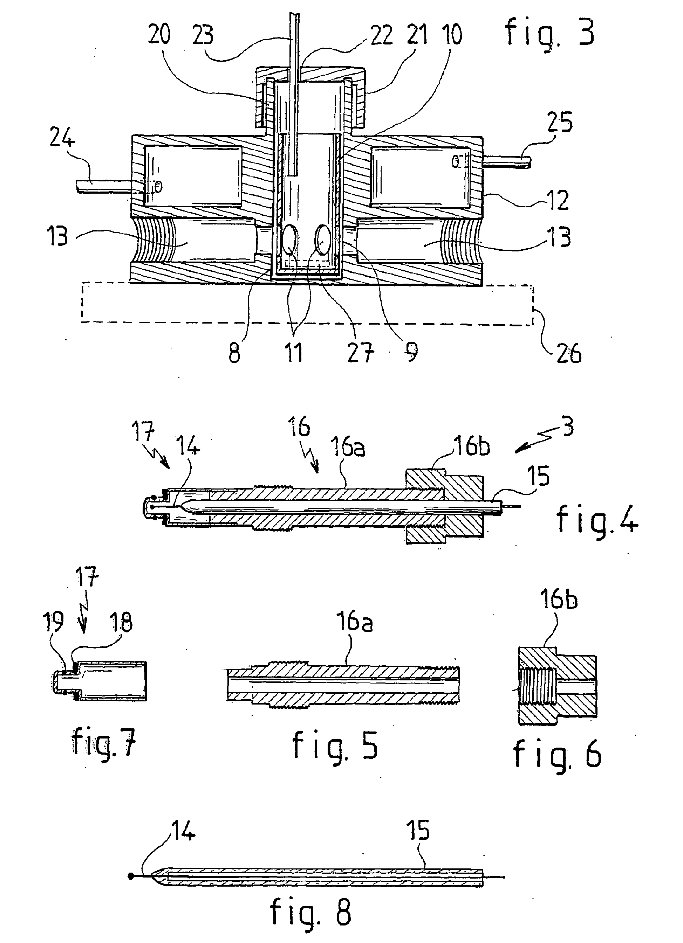

En référence aux figures 2 et 3, la cellule de mesure 1 comporte un puits vertical 8 de section transversale circulaire, ouvert à son extrémité supérieure et fermé à son extrémité inférieure, obtenu dans du polychlorure de vinyle (PVC), muni sur sa paroi latérale, à proximité de son extrémité inférieure, de trous 9 uniformément répartis autour du puits 8 pour permettre aux sondes 3 de déboucher à l'intérieur du puits 8, lesdites sondes 3 étant solidarisées de manière étanche au puits 8 et s'étendant dans des moyens de support uniformément répartis autour du puits 8 et solidaires de ce dernier. On observera que dans cet exemple particulier de réalisation, l'ensemble des trous 9 pratiqués dans le puits 8 de la cellule de mesure 1 pour le passage des sondes 3 s'étendent à une même hauteur par rapport au fond dudit puits 8 ; néanmoins, il est bien évident que les trous 9 pour le passage des sondes 3 pourront être pratiqués à différentes hauteurs par rapport au fond du puits 8 sans pour autant sortir du cadre de l'invention.

La cellule de mesure 1 comprend, par ailleurs, une cuvette 10 de section transversale circulaire homothétique, de diamètre extérieur légèrement inférieur au diamètre intérieur du puits 8 de la cellule de mesure 1, apte à être introduite dans ledit puits 8 et munie sur sa paroi latérale de trous 11 qui s'étendent au droit des trous 9 du puits 8 lorsque la cuvette 10 est positionnée dans ce dernier pour permettre aux sondes 3 de déboucher à l'intérieur de ladite cuvette 10 dans laquelle sont placés les micro-organismes à analyser. Cette cuvette 10 est obtenue dans un matériau métallique qui ne peut pas être magnétisé, tel que de l'inox par exemple pour permettre son nettoyage et sa stérilisation notamment.

Il est bien évident que la cuvette 10 pourra être obtenue dans tout autre matériaux amagnétique susceptible de subir une stérilisation sans détérioration, sans pour autant sortir du cadre de l'invention.

En référence aux figures 2 et 3, les moyens de support des sondes consistent en un tronçon de tube creux vertical cylindrique 12, fermé à ses extrémités supérieure et inférieure, le puits 8 s'étendant coaxialement de telle sorte que l'extrémité supérieure ouverte du puits 8 soit confondue avec l'extrémité supérieure du tube cylindrique 12. Le tube creux cylindrique 12 formant support des sondes 3 comprend des passages horizontaux 13, de section transversale circulaire, uniformément répartis autour du puits 8 en s'étendant radialement depuis la paroi latérale du tube formant support 12 jusqu'à la paroi latérale du puits 8, lesdits passages horizontaux 13 étant aptes à recevoir les sondes 3.

Chaque sonde 3, en référence aux figures 4 à 8 est constituée d'une électrode 14, s'étendant dans un tube en verre fermé 15 en faisant saillie à ses extrémités distale et proximale. On entend par extrémité distale de l'électrode 14 l'extrémité qui débouche à l'intérieur de la cuvette 10 positionnée à l'intérieur du puits 8 de la cellule de mesure 1 et par extrémité proximale, l'extrémité de l'électrode 14 qui fait saillie du tube cylindrique de support 12 des électrodes en s'étendant radialement vers l'extérieur et qui est connectée aux moyens de mesure 4 et aux moyens de traitement 5 des signaux électriques émis par ladite électrode 14. Le tube en verre 15 dans lequel s'étend l'électrode 14 est rempli d'une solution et il est enveloppé par un fourreau 16 comprenant deux parties, une partie principale 16a globalement cylindrique dont le diamètre extérieur est tout juste inférieur au diamètre intérieur des passages 13 et une seconde partie 16b globalement cylindrique apte à venir s'emboîter à l'extrémité proximale de la partie 16a du fourreau 16 et de diamètre extérieur supérieur au diamètre intérieur des passages 13, lesdites parties 16a et 16b du fourreau 16 étant obtenues dans un matériau élastiquement déformable afin de permettre le blocage de la sonde 3 dans le passage 13, lorsqu'elle est introduite dans ledit passage 13. Par ailleurs, l'extrémité distale de l'électrode 14 est coiffée par une membrane 17 creuse et rigide, apte à s'emboîter à l'extrémité distale de la première partie 16a du fourreau 16 enveloppant le tube en verre 15 dans lequel s'étend l'électrode 14.

Il va de soi que la membrane 17 peut être vissée à l'extrémité distale de la première partie 16 a du fourreau 16 qui comprend à cet effet un pas de vis, sans pour autant sortir du cadre de l'invention.

Cette membrane 17 de forme globalement cylindrique comprend à son extrémité libre un rétrécissement de sa section munie de joints toriques 18 et 19 aptes à prendre appui respectivement sur la paroi extérieure du puits 8 et sur l'arrête des trous 11 pratiqués dans la cuvette 10 introduite dans le puits 8 de la cellule de mesure 1 afin d'assurer l'étanchéité de la cuvette 10.

De manière particulièrement avantageuse, le dispositif comprend une sonde 3 comprenant une électrode rendue sélective au salicylate pour mesurer le pH interne (pHin)des micro-organismes qui permet de déduire le gradient ΔpH des micro-organismes qui est égal à la différence entre le pH extérieur et le pH interne , c'est-à-dire :

$$\Delta pH = pH_{in} - pH_{ext}$$

La sonde pour la mesure du $pH_{in}$ est constituée d'une électrode obtenue dans du platine chloruré s'étendant dans un tube en verre fermé en faisant saillie à ses extrémités distales et proximales, ledit tube en verre 15 étant rempli d'une solution de salicylate qui présente une concentration de 2mM et l'extrémité distale de l'électrode 14 étant coiffée par une membrane 17 de polychlorure de vinyle (PVC) traitée au tétraheptylammonium iodure. Le pH extérieur est mesuré à l'aide d'une sonde 3 classiquement constituée d'une électrode dite combinée, c'est-à-dire une électrode comprenant une seconde électrode de référence, obtenue dans du platine s'étendant dans un tube en verre 15 rempli d'une solution d'argent, de chlorure d'argent et de KCel saturé, l'extrémité distale de l'électrode 14 étant coiffée par une membrane 17 en verre laissant passer les ions H3O+.

Le dispositif comprend, par ailleurs, une sonde 3 comprenant une électrode rendue sélective au tétraphényl phosphonium (TPP⁺) pour mesurer le potentiel de membrane communément noté ΔΨ. Cette sonde pour la mesure du potentiel de membrane ΔΨ est constituée d'une électrode 14 obtenue dans du platine chloruré s'étendant dans un tube en verre 15 fermé en faisant saillie à ses extrémités distale et proximale, ledit tube 15 étant rempli d'une solution de tétraphénylphosphonium (TPP⁺) et l'extrémité distale de l'électrode 14 étant coiffée par une membrane 17 de PVC traitée au tétraphénylborure. Afin de permettre la mesure du potentiel de membrane ΔΨ et du $pH_{in}$, le dispositif comprend une sonde 3 de référence dite électrode au calomel constitué d'une électrode 14 obtenue dans du mercure recouvert de calomel $Hg_2Cl_2$ s'étendant dans un tube 15 en verre fermé en faisant saillie à ses extrémités distales et proximales, ledit tube en verre 15 étant rempli d'une solution de chlorure de potassium saturé de calomel. Par ailleurs, le dispositif peut avantageusement comprendre une sonde 3 pour la mesure du potentiel redox $E_h$ ou bien encore une sonde 3 comprenant une électrode pour mesurer l'oxygène dissous.

De manière particulièrement avantageuse, en référence aux figures 1 et 3, l'extrémité supérieure du puits 8 de la cellule de mesure 1 comprend un col 20 apte à être coiffé par un bouchon 21, obtenu dans du caoutchouc ou du PVC par exemple, fermant de manière étanche le puits 8. De plus, le bouchon 21 comprend avantageusement des orifices 22 dans lesquels s'étendent des conduites obturables pour permettre l'introduction et/ou le retrait de matières à l'intérieur de la cuvette 10. Ces conduites 23 permettent ainsi l'échappement de gaz produits par les micro-organismes ou bien encore l'introduction ou le prélèvement de micro-organismes dans la cuvette 10 ou bien encore l'introduction d'un gaz à l'intérieur de la cuvette 10 afin de permettre l'étude des micro-organismes dans différentes compositions atmosphériques et/ou différentes conditions de pressions.

Il va de soi que le bouchon peut consister dans un bouchon à vis muni dans son fond d'un opercule d'étanchéité apte à prendre appui sur le bord supérieur du col 20, ce dernier comprenant un pas de vis.

Par ailleurs, en référence aux figures 1 à 3, le dispositif comprend avantageusement une conduite d'admission 24 d'un fluide caloporteur tel que de l'eau chaude ou froide débouchant dans l'espace formé entre la paroi latérale du tube cylindrique de support 12 et la paroi latérale du puits 8 de la cellule de mesure 1 et une seconde conduite d'échappement 25 de l'eau diamètralement opposée à la conduite d'admission 24, lesdites conduites d'admission 24 et d'échappement 25 étant connectées, par un circuit 6, à des moyens de régulation thermique 7 comprenant, par exemple, une thermistance et des moyens de contrôle de la température connectés à un thermostat. Afin de permettre le suivi de la température des micro-organismes positionnés dans la cuvette 10 de la cellule de mesure 1, le dispositif comprendra avantageusement une sonde thermostatique débouchant à l'intérieur de ladite cuvette 10.

Afin de permettre l'utilisation de la cellule de mesure 1 en bioréacteur, le dispositif comprend des moyens d'agitation des micro-organismes constitués d'une base 26 représentée en traits pointillés sur la figure 3, sur laquelle prend appui le puits 8 et comportant un aimant tournant ou des bobines alternativement excitées telles que décrites dans le brevet français FR 2.539.053, par exemple, aptes à entraîner en rotation un barreau d'agitation 27 placé au fond de la cuvette 10 du dispositif.

Selon une variante d'exécution particulièrement avantageuse de la cellule de mesure suivant l'invention, la paroi latérale du puits vertical 8 est obtenue dans un matériau thermoconducteur tel que l'inox, le verre ou analogue afin de favoriser le transfert thermique du fluide caloporteur qui est en contact avec la paroi latérale extérieure du puits vertical 8 vers les micro-organismes contenus dans ledit puits 8.

Enfin, il va de soi que les sondes peuvent consister dans toute sonde appropriée et que les exemples que l'on vient de donner ne sont que des illustrations particulières en aucun cas limitatives quant aux domaines d'application de l'invention.

**Revendications**

1. - Dispositif pour la mesure d'activités biologiques et / ou de grandeurs physiologiques telles que le pH intracellulaire, le pH extracellulaire, la force proton motrice, l'activité enzymatique ou analogues, comprenant une cellule dite de mesure (1) pourvue d'une chambre (2) apte à recevoir des cellules à analyser telles que des micro-organismes ou des enzymes par exemple et une ou plusieurs sondes (3) débouchant dans la chambre (2), lesdites sondes (3) étant connectées à des moyens de mesure (4) et des moyens de traitement (5) des signaux électriques émis par les sondes (3) ladite cellule de mesure (1) comportant un puits (8) vertical de section transversale quelconque, ouvert à son extrémité supérieure et fermé à son extrémité inférieure, obtenu dans du polychlorure de vinyle (PVC), muni sur sa paroi latérale de trous (9) uniformément répartis autour du puits (8) pour permettre aux sondes (3) de déboucher à l'intérieur du puits (8), lesdites sondes (3) étant solidarisées de manière étanche au puits (8) et s'étendant dans des moyens de support (12) uniformément répartis autour du puits (8) et solidaires de ce dernier, <①> apte à recevoir une cuvette (10) de section transversale homothétique à la section du puits (8) et munie sur sa paroi latérale de trous (11) qui s'étendent au droit des trous (9) du puits (8) lorsque la cuvette (10) est positionnée dans le puits (8) pour permettre aux sondes (3) de déboucher à l'intérieur de ladite cuvette (10) dans laquelle sont placés les micro-organismes à analyser, ladite cuvette étant réalisée dans un matériau métallique amagnétique susceptible de subir une stérilisation sans détérioration.

2. - Dispositif suivant la revendication précédente **caractérisé en ce que** les moyens de support (12) des sondes (3) sont creux et s'étendent autour du puits (8) de la cellule de mesure (1) afin de permettre la circulation d'un fluide caloporteur autour dudit puits (8), le fluide caloporteur étant procuré par une unité de régulation <①>: **caractérisé en ce que** le dit puits est thermique (7).

3. - Dispositif suivant la revendication 2 **caractérisé en ce que** les moyens de support (12) des sondes (3) consistent en un tronçon de tube creux vertical (12), fermé à ses extrémités supérieure et inférieure, le puits (8) s'étendant coaxialement de telle sorte que l'extrémité supérieure du puits (8) soit confondue avec l'extrémité supérieure du tube (12), et muni de passages horizontaux, uniformément répartis autour du puits (8), s'étendant depuis la paroi latérale du tube (12) jusqu'à la paroi latérale du puits (8) et aptes à recevoir les sondes (3).

4. - Dispositif suivant l'une quelconque des revendications 2 ou 3 **caractérisé en ce que** la paroi latérale du puits vertical (8) est obtenue dans un matériau thermoconducteur afin de favoriser le transfert thermique du fluide caloporteur qui est en contact avec la paroi latérale extérieure du puits vertical (8) vers les micro-organismes contenus dans ledit puits (8).

5. - Dispositif suivant la revendication 4 **caractérisé en ce que** le matériau thermoconducteur consiste en de l'inox.

6. - Dispositif suivant la revendication 4 **caractérisé en ce que** le matériau thermoconducteur consiste en du verre.

7. - Dispositif suivant l'une quelconque des revendications 3 à 6 **caractérisé en ce que** le puits (8), la cuvette (10) et le tube (12) présentent une section transversale circulaire.

8. - Dispositif suivant l'une quelconque des revendications précédentes **caractérisé en ce que** chaque sonde (3) est munie d'un fourreau (16) de section tout juste inférieure à la section des passages (13), obtenu dans un matériau classiquement déformable afin de permettre le blocage de la sonde (3) dans le passage (13) lorsque ladite sonde (3) munie de son fourreau (16) est introduite dans ledit passage (13), les extrémités distale et proximale de la sonde (3) faisant saillie dudit fourreau (16).

9. - Dispositif suivant la revendication 8 **caractérisé en ce que** chaque sonde (3) est constituée d'une électrode (14) s'étendant dans un tube en verre fermé (15) en faisant saillie à ses extrémités distale et proximale, ledit tube en verre (15) étant rempli d'une solution et l'extrémité distale de l'électrode (14) étant coiffée par une membrane (17).

10. - Dispositif suivant l'une quelconque des revendications précédentes **caractérisé en ce qu'**il comprend une sonde (3) comprenant une électrode (14) rendue sélective au salicylate pour mesurer le gradient $\Delta$pH des micro-organismes.

11. - Dispositif suivant la revendication 10 **caractérisé en ce que** la sonde pour la mesure du gradient $\Delta$pH$\Psi$ est constituée d'une électrode (14) obtenue dans du platine chloruré s'étendant dans un tube en verre fermé (15) en faisant saillie à ses extrémités distale et proximale ledit tube en verre (15) étant rempli d'une solution de salicylate et l'extrémité distale de l'électrode (14) étant coiffée par une membrane de polychlorure de vinyle (PVC) traitée au tétraheptylammonium iodure.

12. - Dispositif suivant la revendication 11 **caractérisé en ce que** la solution de salicylate présente une concentration de 2mM.

13. - Dispositif suivant l'une quelconque des revendications précédentes **caractérisé en ce qu'**il comprend une sonde (3) comprenant une électrode rendue sélective au tetraphénylphosphonium (TPP$^+$) pour mesurer le potentiel de membrane $\Delta\Psi$.

14. - Dispositif suivant la revendication 13 **caractérisé en ce que** la sonde (3) pour la mesure du potentiel de membrane $\Delta\Psi$ est constituée d'une électrode (14) obtenue dans du platine chloruré s'étendant dans un tube en verre (15) fermé en faisant saillie à ses extrémités distale et proximale, ledit tube en verre (15) étant rempli d'une solution de tetraphéenylphosphonium (TPP$^+$) et l'extrémité distale de l'électrode (14) étant coiffée par une membrane (17) de PVC traitée au tetraphénylborure.

15. - Dispositif suivant l'une quelconque des revendications précédentes **caractérisé en ce qu'**il comprend une sonde (3) de référence dite électrode au "calomel" constituée d'une électrode (14) obtenue dans du mercure recouvert de calomel (Hg$_2$Cl$_2$) s'étendant dans un tube en verre (15) fermé en faisant saillie à ses extrémités distale et proximale, ledit tube en verre (15) étant rempli d'une solution de chlorure de potassium saturé de calomel.

16. - Dispositif suivant l'une quelconque des revendications précédentes **caractérisé en ce qu'**il comprend une sonde (3) pour la mesure du pH.

17. - Dispositif suivant l'une quelconque des revendications précédentes **caractérisé en ce qu'**il comprend une sonde (3) pour la mesure du potentiel redox Eh.

18. - Dispositif suivant l'une quelconque des revendications précédentes **caractérisé en ce que** l'extrémité supérieure du puits (8) comprend un col (20) apte à être coiffé par un bouchon (21) fermant de manière étanche le puits (8).

19. - Dispositif suivant la revendication 18 **caractérisé en ce que** le bouchon (21) comprend au moins un orifice (22) obturable pour permettre l'introduction et/ou le retrait de matière à l'intérieur du puits (8).

20. - Dispositif suivant l'une quelconque des revendications précédentes **caractérisé en qu'**il comprend des moyens d'agitation constitués d'une base (26) sur laquelle prend appui le puits (8) et comportant un aimant tournant ou des bobines alternativement excitées aptes à entraîner en rotation un barreau d'agitation (27) placé au fond de la cuvette (10) du dispositif.

**Claims**

1. A device for measuring biological activities and/or physiological magnitudes such as intracellular pH, extracellular pH, motor proton force, enzymatic activity or similar, comprising a measuring cell (1) provided with a chamber (2) for receiving cells to be analysed, such as micro-organisms or enzymes for example and one or more probes (3) entering the chamber (2), said probes (3) being connected to measuring means (4) and processing means (5) of electrical signals emitted by the probes (3), said measuring cell (1) comprising a vertical well (8) of any transversal cross-section, open at its upper end and closed at its lower end, made of polyvinyl chloride (PVC), fitted on its side wall with holes (9) distributed uniformly around the well (8) to allow the probes (3) to enter the interior of the well (8), said probes (3) being tightly integral with the well (8) and extending into support means (12) distributed uniformly around the well (8) and integral with the latter, **characterised in that** said well is suitable for receiving a cuvette (10) of transversal cross-section similar to the cross-section of the well (8) and fitted on its side wall with holes (11) which extend to the right of the holes (9) of the well (8) when the cuvette (10) is positioned in the well (8) to allow the probes (3) to enter the interior of said cuvette (10) in which are placed the micro-organisms to be analysed, said cuvette being made from an amagnetic metallic material suitable for undergoing sterilization without deterioration.

2. The device as claimed in the preceding claim, **characterised in that** support means (12) of the probes (3) are hollow and extend around the well (8) of the measuring cell (1) to allow circulation of a coolant fluid around said well (8), the coolant fluid being procured by a thermal regulation unit (7).

3. The device as claimed in Claim 2, **characterised in that** support means (12) of the probes (3) consist of a section of hollow vertical tube (12), closed at its upper and lower ends, the well (8) extending coaxially such that the upper end of the well (8) is joined to the upper end of the tube (12), and fitted with horizontal passages, uniformly distributed around the well (8), extending from the side wall of the tube (12) to the side wall of the well (8) and suitable for receiving the probes (3).

4. The device as claimed in any one of Claims 2 or 3, **characterised in that** the side wall of the vertical well (8) is made of a thermoconductive material so as to favour thermal transfer of the coolant fluid which is in contact with the outer side wall of the vertical well (8) to the micro-organisms contained in said well (8).

5. The device as claimed in Claim 4, **characterised in that** the thermoconductive material comprises stainless steel.

6. The device as claimed in Claim 4, **characterised in that** the thermoconductive material comprises glass.

7. The device as claimed in any one of Claims 3 to 6, **characterised in that** the well (8), the cuvette (10) and the tube (12) have a circular transversal cross-section.

8. The device as claimed in any one of the preceding claims, **characterised in that** each probe (3) is fitted with a casing (16) of cross-section slightly smaller than the cross-section of the passages (13), made of a material classically deformable to enable blockage of the probe (3) in the passage (13) when said probe (3) fitted with its casing (16) is introduced into said passage (13), the distal and proximal ends of the probe (3) projecting from said casing (16).

9. The device as claimed in Claim 8, **characterised in that** each probe (3) is constituted by an electrode (14) extending in a closed glass tube (15) by projecting at its distal and proximal ends, said glass tube (15) being filled with a solution and the distal end of the electrode (14) being capped by a membrane (17).

10. The device as claimed in any one of the preceding claims, **characterised in that** it comprises a probe (3) comprising an electrode (14) rendered selective to salicylate for measuring the $\Delta$pH gradient of the microorganisms.

11. The device as claimed in Claim 10, **characterised in that** the probe for measuring the $\Delta$pH$\psi$ is gradient constituted by an electrode (14) made of platinum chloride extending in a closed glass tube (15) by projecting at its distal and proximal ends said glass tube (15) being filled with a salicylate solution and the distal end of the electrode (14) being capped by a membrane of polyvinyl chloride (PVC) treated with tetraheptyl ammonium iodide.

12. The device as claimed in Claim 11, **characterised in that** the salicylate solution has a concentration of 2mM.

13. The device as claimed in any one of the preceding claims, **characterised in that** it comprises a probe (3) comprising an electrode rendered selective to tetraphenyl phosphonium (TPP$^+$) for measuring the membrane potential $\Delta\psi$.

14. The device as claimed in Claim 13, **characterised**

**in that** the probe (3) for measuring the membrane potential $\Delta\psi$ is constituted by an electrode (14) made of platinum chloride extending in a closed glass tube (15) by projecting at its distal and proximal ends, said glass tube (15) being filled with a solution of tetraphenyl phosphonium (TPP$^+$) and the distal end of the electrode (14) being capped by a membrane (17) of PVC treated with tetraphenyl boride.

15. The device as claimed in any one of the preceding claims, **characterised in that** it comprises a reference probe (3) known as a "calomel" electrode constituted by an electrode (14) made of mercury coated in calomel (Hg$_2$Cl$_2$) extending in a closed glass tube (15) by projecting at its distal and proximal ends, said glass tube (15) being filled with a solution of potassium chloride saturated with calomel.

16. The device as claimed in any one of the preceding claims, **characterised in that** it comprises a probe (3) for measuring the pH.

17. The device as claimed in any one of the preceding claims, **characterised in that** it comprises a probe (3) for measuring the potential Eh redox.

18. The device as claimed in any one of the preceding claims, **characterised in that** the upper end of the well (8) comprises a neck (20) to be capped by a stopper (21) tightly closing the well (8).

19. The device as claimed in Claim 18 well (8) **characterised in that** the stopper (21) comprises at least a shut-off orifice (22) to allow introduction and/or the removal of material inside the well (8).

20. The device as claimed in any one of the preceding claims, **characterised in that** it comprises agitation means constituted by a base (26) on which the well (8) is supported, and comprising a revolving magnet or alternatively excited bobbins suitable for rotating an agitation rod (27) placed at the bottom of the cuvette (10) of the device.

## Patentansprüche

1. Vorrichtung zur Messung biologischer Aktivitäten und/oder physiologischer Parameter wie intrazellulärer pH, extrazellulärer pH, protonenmotorische Kraft, enzymatische Aktivität oder ähnliche, eine genannte Messzelle (1) umfassend, die mit einer Kammer (2) ausgestattet ist, die in der Lage ist, die zu analysierenden Zellen wie zum Beispiel Mikroorganismen oder Enzyme aufzunehmen, und eine oder mehrere in die Kammer (2) mündende Sonden (3), wobei die besagten Sonden (3) an Messmittel (4) und Mittel (5) zur Verarbeitung der von den Sonden (3) ausgesendeten elektrischen Signale angeschlossen sind, wobei die besagte Messzelle (1) eine vertikale Vertiefung (8) beliebigen Querschnitts umfasst, die an ihrem oberen Ende geöffnet und an ihrem unteren Ende geschlossen ist, aus Polyvinylchlorid (PVC) hergestellt, an ihrer Seitenwand mit Öffnungen (9) versehen ist, die um die Vertiefung (8) gleichmäßig verteilt sind, um den Sonden (3) zu gestatten, in die Vertiefung (8) zu münden, wobei die besagten Sonden (3) mit der Vertiefung (8) dicht verbunden sind und sich in Trägermittel (12) erstrekken, die um die Vertiefung (8) gleichmäßig verteilt und mit dieser verbunden sind, **dadurch gekennzeichnet, dass** die besagte Vertiefung der Lage ist, eine Küvette (10) mit einem Querschnitt aufzunehmen, der zum Querschnitt der Vertiefung (8) homothetisch ist, und an ihrer Seitenwand mit Öffnungen (11) versehen ist, die sich bei den Öffnungen (9) der Vertiefung (8) erstrecken, wenn die Küvette (10) in der Vertiefung (8) positioniert ist, um den Sonden (3) zu gestatten, in die besagte Küvette (10) zu münden, in der die zu analysierenden Mikroorganismen platziert sind, wobei die besagte Küvette aus einem unmagnetischen metallischen Werkstoff besteht, der ohne Beschädigung sterilisierbar ist.

2. Vorrichtung nach vorangehendem Anspruch, **dadurch gekennzeichnet, dass** die Trägermittel (12) der Sonden (3) hohl sind und sich um die Vertiefung (8) der Messzelle (1) herum erstrecken, um die Zirkulation eines Wärmeträgerfluids um die besagte Vertiefung (8) zu gestatten, wobei das Wärmeträgerfluid von einer Einheit zur thermischen Regulierung (7) bereitgestellt wird.

3. Vorrichtung nach Anspruch 2, **dadurch gekennzeichnet, dass** die Trägermittel (12) der Sonden (3) aus einem vertikalen hohlen Rohrabschnitt (12) bestehen, der an seinem oberen und unteren Ende geschlossen ist, wobei sich die Vertiefung (8) koaxial derart erstreckt, dass das obere Ende der Vertiefung (8) mit dem oberen Ende (12) des Rohrs übereinstimmt, und mit horizontalen Durchgängen ausgestattet ist, die gleichmäßig um die Vertiefung (8) verteilt sind, sich von der Seitenwand des Rohrs (12) bis zur Seitenwand der Vertiefung (8) erstrekken und in der Lage sind, die Sonden (3) aufzunehmen.

4. Vorrichtung nach einem der Ansprüche 2 oder 3, **dadurch gekennzeichnet, dass** die Seitenwand der vertikalen Vertiefung (8) aus einem thermisch leitenden Werkstoff hergestellt ist, um den thermischen Transfer des Wärmeträgerfluids, das mit der äußeren Seitenwand der vertikalen Vertiefung (8) in Kontakt ist, zu den in der besagten Vertiefung (8) enthaltenen Mikroorganismen zu fördern.

5. Vorrichtung nach Anspruch 4, **dadurch gekenn-**

zeichnet, dass der thermisch leitende Werkstoff aus rostfreiem Edelstahl besteht.

6. Vorrichtung nach Anspruch 4, **dadurch gekennzeichnet, dass** der thermisch leitende Werkstoff aus Glas besteht.

7. Vorrichtung nach einem der Ansprüche 3 bis 6, **dadurch gekennzeichnet, dass** die Vertiefung (8), die Küvette (10) und das Rohr (12) einen kreisrunden Querschnitt aufweisen.

8. Vorrichtung nach einem der vorangehenden Ansprüche, **dadurch gekennzeichnet, dass** jede Sonde (3) mit einer Hülse (16) ausgestattet ist, deren Querschnitt etwas kleiner ist als der Querschnitt der Durchgänge (13), die aus einem in klassischer Weise deformierbarem Werkstoff hergestellt ist, um das Blockieren der Sonde (3) in dem Durchgang (13) zu erlauben, wenn die besagte, mit ihrer Hülse (16) versehene Sonde (3) in den besagten Durchgang eingeführt ist, wobei das distale und das proximale Ende der Sonde (3) aus der besagten Hülse (16) hervorstehen.

9. Vorrichtung nach Anspruch 8, **dadurch gekennzeichnet, dass** jede Sonde (3) von einer Elektrode (14) gebildet wird, die sich in ein geschlossenes Glasrohr (15) erstreckt, indem sie an seinem distalen und proximalen Ende hervorsteht, wobei das besagte Glasrohr (15) mit einer Lösung gefüllt ist und das distale Ende der Elektrode (14) von einer Membran (17) bedeckt ist.

10. Vorrichtung nach einem der vorangehenden Ansprüche, **dadurch gekennzeichnet, dass** sie eine Sonde (3) umfasst, die eine Elektrode (14) umfasst, die mit Salicylat selektiv gemacht wurde, um den Gradienten $\Delta pH$ der Mikroorganismen zu messen.

11. Vorrichtung nach Anspruch 10, **dadurch gekennzeichnet, dass** die Sonde zur Messung des Gradienten $\Delta pH$ von einer Elektrode (14) gebildet wird, die aus chlorhaltigem Platin hergestellt ist und sich in ein geschlossenes Glasrohr (15) erstreckt, indem sie an seinem distalen und proximalen Ende hervorsteht, wobei das besagte Glasrohr (15) mit einer Salicylatlösung gefüllt ist und das distale Ende der Elektrode (14) mit einer mit Tetraheptylammoniumiodid behandelten Polyvinylchlorid-Membran (PVC) bedeckt ist.

12. Vorrichtung nach Anspruch 11, **dadurch gekennzeichnet, dass** die Salicylatlösung eine Konzentration von 2 mM aufweist.

13. Vorrichtung nach einem der vorangehenden Ansprüche, **dadurch gekennzeichnet, dass** sie eine Sonde (3) umfasst, die eine Elektrode umfasst, die mit Tetraphenylphosphonium selektiv (TPP$^+$) gemacht wurde, um das Membranpotential $\Delta\Psi$ zu messen.

14. Vorrichtung nach Anspruch 13, **dadurch gekennzeichnet, dass** die Sonde (3) zur Messung des Membranpotentials $\Delta\Psi$ von einer Elektrode (14) gebildet wird, die aus chlorhaltigem Platin hergestellt ist und sich in ein geschlossenes Glasrohr (15) erstreckt, indem sie an seinem distalen und proximalen Ende hervorsteht, wobei das besagte Glasrohr (15) mit einer Tetraphenylphosphoniumlösung (TPP$^+$) gefüllt ist und das distale Ende der Elektrode (14) von einer Membran (17) aus mit Tetraphenylborid behandeltem PVC bedeckt ist.

15. Vorrichtung nach einem der vorangehenden Ansprüche, **dadurch gekennzeichnet, dass** sie eine Referenzsonde (3), "Calomel"-Elektrode genannt, umfasst, die von einer Elektrode (14) gebildet wird, die aus mit Calomel ($Hg_2Cl_2$) bedecktem Quecksilber hergestellt ist und sich in ein geschlossenes Glasrohr (15) erstreckt, indem sie an seinem distalen und proximalen Ende hervorsteht, wobei das besagte Glasrohr (15) mit einer mit Calomel gesättigten Kaliumchloridlösung gefüllt ist.

16. Vorrichtung nach einem der vorangehenden Ansprüche, **dadurch gekennzeichnet, dass** sie eine Sonde (3) zur Messung des pH umfasst.

17. Vorrichtung nach einem der vorangehenden Ansprüche, **dadurch gekennzeichnet, dass** sie eine Sonde (3) zur Messung des Redoxpotentials Eh umfasst.

18. Vorrichtung nach einem der vorangehenden Ansprüche, **dadurch gekennzeichnet, dass** das obere Ende der Vertiefung (8) einen Hals (20) umfasst, der in der Lage ist, von einem Verschluss (21) bedeckt zu werden, der die Vertiefung (8) dicht verschließt.

19. Vorrichtung nach Anspruch 18, **dadurch gekennzeichnet, dass** der Verschluss (21) mindestens eine verschließbare Öffnung (22) umfasst, um das Einführen und/oder Herausnehmen von Material in der Vertiefung (8) zu gestatten.

20. Vorrichtung nach einem der vorangehenden Ansprüche, **dadurch gekennzeichnet, dass** sie Rührmittel umfasst, die von einer Basis (26) gebildet werden, auf der sich die Vertiefung (8) abstützt, einen Drehmagneten oder abwechselnd erregte Spulen umfassend, die in der Lage sind, einen auf dem Boden der Küvette (10) der Vorrichtung platzierten Rührstab rotierend anzutreiben (27).

fig.1

fig.2

fig. 3

fig. 4

fig. 7

fig. 5

fig. 6

fig. 8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 6096275 A
- SU 1656438
- FR 2779525
- FR 2539053